Europäisches Patentamt

European Patent Office

Office européen des brevets

(11) Veröffentlichungsnummer: **0 342 325**
**A2**

(12) **EUROPÄISCHE PATENTANMELDUNG**

(21) Anmeldenummer: **89103880.4**

(22) Anmeldetag: **06.03.89**

(51) Int. Cl.4: **C07K 5/02 , C07D 239/42 ,**
**C07D 233/64 , A61K 37/64 ,**
**A61K 31/395**

Die Bezeichnung der Erfindung wurde geändert (Richtlinien für die Prüfung im EPA, A-III, 7.3).

(30) Priorität: **18.03.88 DE 3809145**

(43) Veröffentlichungstag der Anmeldung:
**23.11.89 Patentblatt 89/47**

(84) Benannte Vertragsstaaten:
**AT BE CH DE ES FR GB IT LI NL SE**

(71) Anmelder: **MERCK PATENT GESELLSCHAFT**
**MIT BESCHRÄNKTER HAFTUNG**
**Frankfurter Strasse 250 Postfach 4119**
**D-6100 Darmstadt(DE)**

(72) Erfinder: **Raddatz, Peter, Dr.**
**Grafenstrasse 37**
**D-6100 Darmstadt(DE)**
Erfinder: **Sombroek, Johannes, Dr.**
**Robert-Koch-Strasse 32**
**D-6100 Darmstadt 13(DE)**
Erfinder: **Gante, Joachim, Dr.**
**Stormstrasse 4**
**D-6100 Darmstadt 12(DE)**
Erfinder: **Schmitges, Claus J., Dr.**
**Karolinger Strasse 5**
**D-6114 Gross-Umstadt(DE)**
Erfinder: **Minck, Klaus-Otto, Dr.**
**Büchestrasse 8**
**D-6105 Ober-Ramstadt(DE)**

(54) **Renin inhibierende Aminosäurederivate.**

(57) Neue Aminosäurederivate der Formel I
$$R^1-Z-NR^2-CHR^3-CR^4-(CHR^5)_n-CO-E-Q-Y \qquad I$$
worin
$R^1$, Z, $R^2$, $R^3$, $R^4$, $R^5$, n, E, Q und Y die in Patentanspruch 1 angegebene Bedeutung haben,
sowie ihre Salze hemmen die Aktivität des menschlichen Plasmarenins.

EP 0 342 325 A2

# Aminosäurederivate

Die Erfindung betrifft neue Aminosäurederivate der Formel I

$R^1$-Z-NR$^2$-CHR$^3$-CR$^4$-(CHR$^5$)$_n$-CO-E-Q-Y     I

worin

R$^1$     [R$^6$R$^7$N-C$_m$H$_{2m}$-(T)$_x$-(CO)$_y$-C$_r$H$_{2r}$]-L(R$^8$-C$_p$H$_{2p}$)-CO- oder     [R$^9$-O-(CO)$_z$-C$_m$H$_{2m}$-(T)$_x$-(CO)$_y$-C$_r$H$_{2r}$]-L(R$^8$-C$_p$H$_{2p}$)-CO-,

Z     Abu, Ada, Ala, βAla, Arg, Asn, Asp, Bia, Cal, Dab, Ftr, Gln, Glu, Gly, His, Hph, N(im)-A-His, Ile, Leu, tert.-Leu, Lys, Met, αNal, βNal, Nbg, Nle, Nva, Orn, Phe, Pia, Pro, Pya, Ser, Thr, Tia, Tic, Trp, Tyr oder Val,

E     0 bis 2 peptidartig miteinander verbundene Aminosäurereste ausgewählt aus der Gruppe bestehend aus Abu, Ala, Cal, His, Ile, Leu, Met, Nle, Nva, Phe, Trp, Tyr und Val,

Q     O oder NR$^{10}$ ,

Y     -C$_t$H$_{2t}$-R$^{11}$, -C$_t$H$_{2t}$-R$^{12}$ oder -C$_w$H$_{2w}$-(CR$^{13}$)$_s$-C$_t$H$_{2t}$-R$^{11}$,

T     O oder NR$^{14}$,

L     CH oder N,

R$^2$, R$^5$, R$^9$, R$^{10}$ und R$^{14}$     jeweils H oder A,

R$^3$, R$^8$ und R$^{11}$     jeweils H, A, Ar, Ar-alkyl, Het, Het-alkyl, unsubstituiertes oder ein- oder mehrfach durch A, AO und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

R$^4$ und R$^{13}$     jeweils (H, OH), (H, NH$_2$) oder = O,

R$^6$ und R$^7$     jeweils H, A, -C( = NH)-NH$_2$ oder -C-( = NH)-NHCN,

R$^6$     auch Ac,

R$^{12}$     -SO$_3$H, -SO$_2$NH$_2$, -SO$_2$NHA, -SO$_2$NA$_2$, -NH$_2$, -NHA, -NA$_2$, -NH-C( = NH)-NH$_2$, -NH-C-( = NH)-NHCN, -NH-CO-NH$_2$, -NH-CO-NHA, -NH-CO-NA$_2$, -NH-CS-NH$_2$, -NH-CS-NHA oder -NH-CS-NA$_2$

. n und s     jeweils 1 oder 2,

m, p, r, t und w     jeweils 0, 1, 2, 3, 4, 5, 6, 7, 8, 9 oder 10,

x, y und z     jeweils 0 oder 1

Ar     unsubstituiertes oder ein- oder mehrfach durch A, OA, Hal, CF$_3$, OH, NO$_2$, Hydroxyalkyl, NH$_2$, NHA, NA$_2$, NHAc, SA, SO-A, SO$_2$-A, SO$_2$NH$_2$, SO$_2$NHA, COOH, COOA, CONH$_2$, CN, Aminoalkyl, HAN-alkyl, A$_2$N-alkyl, A$_3$N$^\oplus$alkyl An$^\ominus$ und/oder Guanidinyl-alkyl substituiertes Phenyl oder unsubstituiertes Naphthyl,

Het     einen gesättigten oder ungesättigten 5- oder 6-gliedrigen heterocyclischen Rest mit 1-4 N-, O- und/oder S-Atomen, der mit einem Benzolring kondensiert und/oder ein- oder mehrfach durch A, OA, Hal, CF$_3$, OH, NO$_2$, Carbonylsauerstoff, NH$_2$, NHA, NA$_2$, NHAc, SA, SO-A, SO$_2$-A, SO$_2$NH$_2$, SO$_2$NHA, COOH, COOA, CONH$_2$, CN, NH-SO$_2$-A, Ar, Ar-alkyl, Ar-alkenyl, Hydroxyalkyl und/oder Aminoalkyl substituiert sein kann und/oder dessen N- und/oder S-Heteroatome auch oxydiert sein können,

Hal     F, Cl, Br oder J,

Ac     A-CO-, Ar-CO-, Ar-alkyl-CO-, A-O-CO-, Ar-O-CO-, Ar-alkyl-O-CO- oder A-NH-CO-,

An$^\ominus$     ein Anion, das auch fehlen kann, wenn stattdessen eine in der Verbindung der Formel I enthaltene Carboxygruppe in Form eines Carboxylatanions vorliegt,

-alkyl-     eine Alkylengruppe mit 1-8 C-Atomen und

A     Alkyl mit 1-8 C-Atomen bedeuten,

worin ferner an Stelle einer oder mehrerer -NH-CO-Gruppen auch eine oder mehrere -NA-CO-Gruppen stehen können,

sowie deren Salze.

Ähnliche Verbindungen sind aus der EP-A-249096 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem hemmen sie die Aktivität des menschlichen Plasmarenins. Diese Wirkung kann z.B. nach der Methode von F. Fyhrquist et al., Clin.Chem. 22, 250-256 (1976), nachgewiesen werden. Bemerkenswert ist, daß diese Verbindungen sehr spezifische Hemmer des Renins sind; für die Hemmung anderer Aspartylproteinasen (z.B. Pepsin und Kathepsin D) sind in der Regel etwa 100 bis 1000 mal so hohe Konzentrationen dieser Verbindungen notwendig wie für die Reninhemmung. Die Wirkungen der Verbindungen auf den Blutdruck und/oder auf die Herzfrequenz sowie die Hemmung der Reninaktivität im Blutplasma können ferner an wachen Affen, z.B. weiblichen Affen (Macaca fascicularis) ermittelt werden; dabei können Blutdruck und Herzfrequenz in Anlehnung an die Methode von M.J. Wood et al., J. Hypertension 4, 251-254 (1985) gemessen werden. Zur Stimulierung der Reninaktivität werden die Tiere dabei zweckmäßig mit einem Saluretikum vorbehandelt. Blutproben zur Bestimmung der Plasma-Reninaktivität können durch Punktion der Vena femoralis gewonnen werden.

Die Verbindungen können als Arzneimittelwirkstoffe in der Human- und Veterinärmedizin eingesetzt werden, insbesondere zur Prophylaxe und zur Behandlung von Herz-, Kreislauf- und Gefäßkrankheiten, vor allem von Hypertonie, Herzinsuffizienz und Hyperaldosteronismus. Außerdem können die Verbindungen zu diagnostischen Zwecken verwendet werden, um bei Patienten mit Hypertonie oder Hyperaldosteronismus den möglichen Beitrag der Reninaktivität zur Aufrechterhaltung des pathologischen Zustands zu bestimmen. Solche diagnostische Tests können ausgeführt werden ähnlich wie es in der EP-A-77 028 angegeben ist.

Die vor- und nachstehend aufgeführten Abkürzungen von Aminosäureresten stehen für die Reste -NR'-R"-CO-, in der Regel -NH-CHR-CO- (worin R, R' und R" die für jede Aminosäure bekannte spezifische Bedeutung haben) folgender Aminosäuren:

Abu    2-Aminobuttersäure
Ada    3-(1-Adamantyl)-alanin
AHCH    4S-Amino-3S-hydroxy-6-cyclohexyl-hexansäure
AHCP    4S-Amino-3S-hydroxy-5-cyclohexyl-pentansäure
AHPP    4S-Amino-3S-hydroxy-5-phenyl-pentansäure
Ala    Alanin
βAla    β-Alanin
Arg    Arginin
Asn    Asparagin
Asp    Asparaginsäure
Bia    3-(2-Benzimidazolyl)-alanin
Cal    3-Cyclohexylalanin
Dab    2,4-Diaminobuttersäure
DACH    3S,4S-Diamino-6-cyclohexyl-hexansäure
DACP    3S,4S-Diamino-5-cyclohexyl-pentansäure
DAMH    3S,4S-Diamino-6-methyl-heptansäure
DAPP    3S,4S-Diamino-5-phenyl-pentansäure
Ftr    N-Formyl-tryptophan
Gln    Glutamin
Glu    Glutaminsäure
Gly    Glycin
His    Histidin
N(im)-A-His    in 1- oder 3-Stellung des Imidazolrings durch A substituiertes Histidin
Hph    Homo-phenylalanin ( = 2-Amino-4-phenylbuttersäure)
Ile    Isoleucin
Leu    Leucin
tert.-Leu    tert.-Leucin
Lys    Lysin
Met    Methionin
αNal    3-(α-Naphthyl)-alanin
βNal    3-(β-Naphthyl)-alanin
Nbg    (2-Norbornyl)-glycin
Nle    Norleucin
N-Me-His    N-Methyl-histidin
N-Me-Phe    N-Methyl-phenylalanin
Nva    Norvalin
Orn    Ornithin
Phe    Phenylalanin
Pia    3-(Piperidyl)-alanin [z.B. 2-Pia 3-(2-Piperidyl)-alanin]

Pro    Prolin
Pya    3-(Pyridyl)-alanin [z.B. 3-Pya 3-(3-Pyridyl)-alanin]
Ser    Serin
Sta    Statin
Thr    Threonin
Tia    3-(Thienyl)-alanin [z.B. 2-Tia 3-(2-Thienyl)-alanin]
Tic    1,2,3,4-Tetrahydroisochinolin-1-carbonsäure
Trp    Tryptophan
Tyr    Tyrosin
Val    Valin.

Ferner bedeutet nachstehend:
ADPA    N-2-Amino-5,6-dimethyl-3-pyrazinylmethyl-amid
AMPA    N-4-Amino-2-methyl-5-pyrimidinylmethyl-amid
BDP    2-Benzyl-3-(N-3-dimethylaminopropyl-carbamoyl)-propionyl
BOC    tert.-Butoxycarbonyl
BOM    Benzyloxymethyl
imi-BOM    Benzyloxymethyl in 1-Stellung des Imidazolrings
CBZ    Benzyloxycarbonyl
DCCI    Dicyclohexylcarbodiimid
DMF    Dimethylformamid
DNP    2,4-Dinitrophenyl
imi-DNP    2,4-Dinitrophenyl in 1-Stellung des Imidazolrings
ETOC    Ethoxycarbonyl
FMOC    9-Fluorenylmethoxycarbonyl
HOBt    1-Hydroxybenzotriazol
IPOC    Isopropoxycarbonyl
NDP    2-(1-Naphthylmethyl)-3-(N-3-dimethylaminopropylcarbamoyl)-propionyl
OMe    Methylester
OEt    Ethylester
POA    Phenoxyacetyl.

Sofern die vorstehend genannten Aminosäuren in mehreren enantiomeren Formen auftreten können, so sind vor- und nachstehend, z.B. als Bestandteil der Verbindungen der Formel I, alle diese Formen und auch ihre Gemische (z.B. die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich etwas anderes angegeben ist.

Gegenstand der Erfindung ist ferner ein Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt oder daß man eine Carbonsäure der Formel II

$$R^1\text{-}G^1\text{-OH} \qquad \text{II}$$

worin $G^1$

(a) fehlt,

(b) Z,

(c) Z-W,

(d) Z-W-E$^1$,

(e) Z-W-E und

W -NR$^2$-CHR$^3$-CR$^4$-(CHR$^5$)$_n$-CO- bedeuten

mit einer Aminoverbindung der Formel III

H-G$^2$ III

worin G$^2$

(a) -Z-W-E-Q-Y,

(b) -W-E-Q-Y,

(c) -E-Q-Y-,

(d) -E$^2$-Q-Y,

(e) -NR$^{10}$-Y

E$^1$ + E$^2$ zusammen E bedeuten

umsetzt,

und daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder zur Herstellung einer Verbindung der Formel I, R$^4$ = (H, OH) oder (H, NH$_2$), ein Aminoketosäurederivat der Formel I, R$^4$ = O, reduziert oder reduktiv aminiert und/oder eine Verbindung der Formel I durch Behandeln mit einer Säure in eines ihrer Salze überführt.

Vor- und nachstehend haben die Reste bzw. Parameter R$^1$ bis R$^{14}$, Z, E, Q, Y, T, L, m, n, p, r, s, t, w, x. y, z, Ar, Het, Hal, Ac, An, A, G$^1$, G$^2$, E$^1$, E$^2$ und W die bei den Formeln I, II oder III angegebenen Bedeutungen, falls nicht ausdrücklich etwas anderes angegeben ist.

In den vorstehenden Formeln hat A 1-8, vorzugsweise 1, 2, 3 oder 4 C-Atome. A bedeutet vorzugsweise Methyl, weiterhin Ethyl, Propyl, Isopropyl, Butyl, Isobutyl, sek.-Butyl, oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, Heptyl, Octyl.

Cycloalkyl bedeutet vorzugsweise Cyclopropyl, Cyclobutyl, Cyclopentyl, Cyclohexyl oder Cycloheptyl, aber auch z.B. 1-, 2- oder 3-Methylcyclopentyl, 1-, 2-, 3- oder 4-Methylcyclohexyl.

Dementsprechend bedeutet Cycloalkylalkyl vorzugsweise Cyclopropylmethyl, 2-Cyclopropylethyl, Cyclobutylmethyl, 2-Cyclobutylethyl, Cyclopentylmethyl, 2-Cyclopentylethyl, Cyclohexylmethyl, 2-Cyclohexylethyl, aber auch z.B. 1-, 2- oder 3-Methylcyclopentylmethyl, 1-, 2-, 3- oder 4-Methylcyclohexylmethyl.

Bicycloalkyl bedeutet vorzugsweise 1- oder 2-Dekalyl, 2-Bicyclo[2,2,1]heptyl oder 6,6-Dimethyl-2-bicyclo[3,1,1]heptyl.

Tricycloalkyl bedeutet vorzugsweise 1-Adamantyl.

Hal bedeutet vorzugsweise F, Cl oder Br, aber auch J.

Ac bedeutet vorzugsweise A-CO-, wie Acetyl, Propionyl oder Butyryl, Ar-CO- wie Benzoyl, o-, m- oder p-Methoxy-benzoyl oder 3,4-Dimethoxybenzoyl, A-NH-CO- wie N-Methyl-oder N-Ethylcarbamoyl.

Ar bedeutet vorzugsweise Phenyl, ferner bevorzugt o-, m- oder p-Tolyl, o-, m- oder p-Ethylphenyl, o-, m- oder p-Methoxyphenyl, o-, m- oder p-Fluorphenyl, o-, m- oder p-Chlorphenyl, o-, m- oder p-Bromphenyl, o-, m- oder p-Jodphenyl, o-, m- oder p-Trifluormethylphenyl, o-, m-oder p-Hydroxyphenyl, o-, m- oder p-Sulfamoylphenyl, 2,3-, 2,4-, 2,5-, 2,6-, 3,4- oder 3,5-Dimethoxyphenyl, 3,4,5-Trimethoxyphenyl, o-, m- oder p-Aminophenyl, o-, m-oder p-Aminomethylphenyl, o-, m- oder p-Dimethylaminomethylphenyl, o-, m- oder p-Guanidinomethylphenyl, 1- oder 2-Naphthyl.

Dementsprechend bedeutet Ar-alkyl vorzugsweise Benzyl, 1- oder 2-Phenylethyl, o-, m- oder p-Methylbenzyl, 1-oder 2-o-, -m- oder -p-Tolylethyl, o-, m- oder p-Ethylbenzyl, 1- oder 2-o-, -m- oder -p-Ethylphenylethyl, o-, m- oder p-Methoxybenzyl, 1- oder 2-o-, -m- oder -p-Methoxyphenylethyl, o-, m- oder p-Fluorbenzyl, 1- oder 2-o-, -m- oder -p-Fluorphenylethyl, o-, m- oder p-Chlorbenzyl, 1-oder 2-o-, -m- oder -p-Chlorphenylethyl, o-, m- oder p-Brombenzyl, 1- oder 2-o-, -m- oder -p-Bromphenylethyl, o-, m- oder p-Jodbenzyl, 1- oder 2-o-, -m-oder -p-Jodphenylethyl, o-, m- oder p-Trifluormethylbenzyl, o-, m- oder p-Hydroxybenzyl, 2,3-, 2,4-, 2,5-, 2,6-, 3,4- oder 3,5-Dimethoxybenzyl, 3,4,5-Trimethoxybenzyl, o-, m- oder p-Aminobenzyl, o-, m- oder p-Aminomethylbenzyl, o-, m- oder p-Dimethylaminomethylbenzyl, o-, m- oder p-Guanidinomethylbenzyl, 1- oder 2-Naphthylmethyl.

Het ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4-oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4-oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 2,1,5-Thiadiazol-3- oder -4-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6-oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnolyl, 2-, 4-, 5-, 6- 7- oder 8-Chinazolyl. Die heterocyclischen Reste können auch teilweise oder vollständig hydriert sein. Het kann also z.B. auch bedeuten 2,3-Dihydro-2-, -3-, -4- oder -5-furyl, 2,5-Dihydro-2-, -3-, -4- oder -5-furyl, Tetrahydro-2- oder -3-furyl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3-oder -4-pyridyl, 1,2,3,4-Tetrahydro-1-, -2-, -3-, -4-, -5- oder -6-pyridyl, 1,2,3,6-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder 8-isochinolyl.

Die heterocyclischen Reste können auch wie angegeben substituiert sein. Het kann z.B. bevorzugt auch bedeuten: 2-Amino-4-thiazolyl, 4-Carboxy-2-thiazolyl, 4-Carbamoyl-2-thiazolyl, 4-(2-Aminoethyl)-2-thiazolyl, 4-Amino-2-methyl-5-pyrimidinyl, 2-Amino-5,6-dimethyl-3-pyrazinyl, 4-Carbamoylpiperidino, ferner z.B. 3-, 4- oder 5-Methyl-2-furyl, 2-, 4- oder 5-Methyl-3-furyl, 2,4-Dimethyl-3-furyl, 5-Nitro-2-furyl, 5-Styryl-2-furyl, 3-, 4- oder 5-Methyl-2-thienyl, 2-, 4- oder 5-Methyl-3-thienyl, 3-Methyl-5-tert.-butyl-2-thienyl, 5-Chlor-2-thienyl, 5-Phenyl-2- oder -3-thienyl, 1-, 3-, 4- oder 5-Methyl-2-pyrrolyl, 1-Methyl-4- oder -5-nitro-2-pyrrolyl, 3,5-Dimethyl-4-ethyl-2-pyrrolyl, 4-Methyl-5-pyrazolyl, 5-Methyl-3-isoxazolyl, 3,4-Dimethyl-5-isoxazolyl, 4- oder 5-Methyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 3-, 4-, 5- oder 6-Chlor-2-pyridyl, 2-, 4-, 5- oder 6-Chlor-3-pyridyl, 2- oder 3-Chlor-4-pyridyl, 2,6-Dichlorpyridyl, 2-Hydroxy-3-, -4-, -5- oder -6-pyridyl (= 1H-2-Pyridon-3-, -4-, -5- oder -6-yl), 5-Phenyl-1H-2-pyridon-3-yl, 5-p-Methoxyphenyl-1H-2-pyridon-3-yl, 2-Methyl-3-hydroxy-4-hydroxymethyl-5-pyridyl, 2-Hydroxy-4-amino-6-methyl-3-pyridyl, 3-N'-Methylureido-1H-4-pyridon-5-yl, 4-Methyl-2-pyrimidinyl, 4,6-Dimethyl-2-pyrimidinyl, 2-, 5- oder 6-Methyl-4-pyrimidinyl, 2,6-Dimethyl-4-pyrimidinyl, 2,6-Dihydroxy-4-pyrimidinyl, 5-Chlor-2-methyl-4-pyrimidinyl, 3-Methyl-2-benzofuryl, 2-Ethyl-3-benzofuryl, 7-Methyl-2-benzothienyl, 1-, 2-, 4-, 5-, 6-oder 7-Methyl-3-indolyl, 1-Methyl-5- oder -6-benzimidazolyl, 1-Ethyl-5- oder -6- benzimidazolyl, 3-, 4-, 5-, 6-, 7- oder 8-Hydroxy-2-chinolyl, 2-Oxo-pyrrolidino, 2-Oxo-piperidino, 2,5-Dioxopyrrolidino, 3-Benzyl-2,5-dioxopyrrolidino.

Z bedeutet vorzugsweise Gly, βAla oder His, weiterhin bevorzugt Abu, Ada, Asn, Bia, Cal, Gln, Gly, N(im)-Methyl-His, Leu, αNal, βNal, Nle, Phe, Pia, Pya, Tia, Trp oder Tyr.

Die Parameter n und s sind vorzugsweise jeweils 1, m, p und r jeweils 0,1,2,3,4,5,od.6 ; im einzelnen ist m bevorzugt 2, 3, 4, 5 oder 6 (insbesondere 4, 5 oder 6), p bevorzugt 0 und r bevorzugt 1 oder 2. Die Gruppen $C_tH_{2t}$ und $C_wH_{2w}$ sind vorzugsweise jeweils $-CH_2-$, $-(CH_2)_2-$, $-CH(CH_3)-$, $-CH(Isobutyl)-$ oder $-CH(sek.-Butyl)-$; die Gruppe $C_tH_{2t}$ kann bevorzugt auch fehlen (t = 0). Die Parameter x, y und z sind vorzugsweise jeweils 1.

T ist vorzugsweise $NR^{14}$ insbesondere NH, ferner $N(CH_3)$ oder $N(C_2H_5)$.

L ist vorzugsweise CH.

Dementsprechend ist $R^1$ vorzugsweise $(R^6R^7N-C_mH_{2m}-NR^{14}-CO-C_rH_{2r})-L(R^8-C_pH_{2p})-CO-$, andererseits vorzugsweise $(R^6R^7N-C_mH_{2m}-T-CO-C_rH_{2r})-CH(R^8-C_pH_{2p})-CO-$, insbesondere bevorzugt $(R^6R^7N-C_mH_{2m}-NR^{14}-CO-C_rH_{2r})-CH(R^8-C_pH_{2p})-CO-$. Engere bevorzugte Bedeutungen von $R^1$ sind $(R^6R^7N-C_mH_{2m}-NH-CO-C_rH_{2r})-CH(R^8-C_pH_{2p})-CO-$, $[R^6R^7N-(CH_2)_m-NH-CO-(CH_2)_r]-CH[R^8-(CH_2)_p]-CO-$, $[R^6R^7N-(CH_2)_m-NH-CO-(CH_2)_r]-CH[Ar-(CH_2)_p]-CO-$, $[R^6R^7N-(CH_2)_m-NH-CO-(CH_2)_r]-CH(ArCH_2)-CO-$, $[R^6R^7N-(CH_2)_m-NH-CO-(CH_2)]-CH[Ar-(CH_2)_p]-CO-$ und $[R^6R^7N-(CH_2)_m-NH-CO-(CH_2)]-CH[ArCH_2]-CO-$, worin m bevorzugt 2 oder 3 und Ar bevorzugt Phenyl oder 1- oder 2-Naphthyl bedeuten. Besonders bevorzugte Reste $R^1$ sind BDP und NDP.

$R^2$, $R^5$, $R^6$, $R^7$, $R^{10}$ und $R^{14}$ bedeuten vorzugsweise H oder Methyl, ferner Ethyl, Propyl, Isopropyl, Butyl oder Isobutyl, $R^6$ auch bevorzugt $-C(=NH)-NH_2$ oder $-C(=NH)-NH-CN$; in diesen beiden letzten Fällen ist $R^7$ bevorzugt gleichzeitig H. Die Gruppe $R^6R^7N-$ bedeutet dementsprechend vorzugsweise Dimethylamino, ferner Amino, Methylamino, Ethylamino, Diethylamino, Guanidino oder N'-Cyanguanidino. Falls n = 2 ist, können die beiden Reste $R^5$ gleich oder voneinander verschieden sein; vorzugsweise ist in diesem Fall der eine Rest $R^5$ H; der andere A, insbesondere Isopropyl, die Gruppe $-(CHR^5)_n$ bevorzugt $-CH_2-CHA-$, insbesondere $-CH_2-CH(Isopropyl)-$.

$R^3$ bedeutet vorzugsweise Cyclohexylmethyl, ferner bevorzugt A, insbesondere Methyl, Ethyl, Propyl, Isopropyl, Butyl, Isobutyl, sek.-Butyl, Pentyl, Isopentyl (3-Methylbutyl) oder 2-Methylbutyl, Phenyl, Benzyl, p-Chlorbenzyl, 2-Cyclohexylethyl, Bicyclo[2,2,1]heptyl-2-methyl oder 6,6-Dimethylbicyclo[3,1,1]heptyl-2-methyl.

$R^4$ und $R^{13}$ sind jeweils vorzugsweise (H, OH).

R$^8$ ist bevorzugt Ar, insbesondere Phenyl, o-, m- oder p-Methoxyphenyl oder 1- oder 2-Naphthyl.

R$^9$ bedeutet vorzugsweise H.

Die Gruppe W bedeutet vorzugsweise -NH-CHR$^3$-CHOH-CH$_2$-CO-, insbesondere AHCP, AHCH, Sta oder AHPP. Die Gruppe W bedeutet ferner bevorzugt -NH-CHR$^3$-CH(NH$_2$)-CH$_2$-CO-, insbesondere DACP, DACH, DAMH oder DAPP.

Die Gruppe W besitzt mindestens ein chirales Zentrum. In den Gruppen R$^1$ bis R$^5$, Z, E, Q und Y können weitere chirale Zentren vorhanden sein. Die Verbindungen der Formel I können daher in verschiedenen - optisch-inaktiven oder optisch-aktiven - Formen vorkommen. Die Formel I umschließt alle diese Formen. Falls W -NH-CHR$^3$-CR$^4$-CH$_2$-CO- mit R$^4$ = (H, OH) oder (H, NH$_2$) bedeutet, sind die 3S-Hydroxy-4S-amino-Enantiomeren bzw. 3S,4S-Diamino-Enantiomeren bevorzugt. Die Abkürzungen AHCP, AHCH, Sta, AHPP, DACP, DACH, DAMH und DAPP beziehen sich immer auf die 3S,4S-Formen.

Die oben erwähnten substituierten Cycloalkyl- und Phenylgruppen tragen vorzugsweise 1 bis 3, insbesondere 1 oder 2 Substituenten.

E bedeutet vorzugsweise einen der genannten Aminosäurereste, insbesondere Ile oder Leu; ferner ist E bevorzugt abwesend oder bedeutet bevorzugt Abu, Cal, Met, Nle, Nva, Phe oder Val.

Q ist vorzugsweise NR$^{10}$, insbesondere NH oder N(CH$_3$).

Y ist vorzugsweise -C$_t$H$_{2t}$-R$^{11}$ oder C$_t$H$_{2t}$-R$^{12}$, insbesondere -CH$_2$R$^{11}$, -CH$_2$R$^{12}$ oder -CH$_2$CH$_2$R$^{12}$. Dabei bedeutet R$^{11}$ vorzugsweise H, A, Ar oder Het, im einzelnen bevorzugt H, Alkyl mit 3-5 C-Atomen, Phenyl, o-, m- oder p-Aminomethylphenyl, o-, m- oder p-Guanidinomethylphenyl, o-, m-oder p-Dialkylaminomethylphenyl wie o-, m- oder p-Dimethylaminomethylphenyl, 2-, 3- oder 4-Pyridyl, 2-Hydroxy-4,6-dimethyl-3-pyridyl, 4-Amino-2-methyl-5-pyrimidinyl oder 2-Amino-5,6-dimethyl-3-pyrazinyl. R$^{12}$ bedeutet vorzugsweise -SO$_3$H, -SO$_2$NH$_2$ -NA$_2$, -NA$_3^+$ An$^-$, -NH-C(=NH)-NH$_2$, -NH-CO-NHA oder -NH-CS-NHA, wobei A bevorzugt CH$_3$ ist.

Einige besonders bevorzugte Bedeutungen der Gruppe Q-Y sind -NH-CH$_2$-(4-amino-2-methyl-5-pyrimidinyl) ("AMPA"), -NH-CH$_2$-(2-amino-5,6-dimethyl-3-pyrazinyl) ("ADPA") und -NH-CH$_2$-(3-pyridyl), ferner -NH-A.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ii ausgedrückt werden, die der Formel I entsprechen, worin jedoch

in Ia

R$^1$  (R$^6$R$^7$N-C$_m$H$_{2m}$-NH-CO-C$_r$H$_{2r}$)-CH-(Ar-

C$_p$H$_{2p}$)-CO-,

R$^6$R$^7$N  Amino, Methylamino, Dimethylamino, Ethylamino, Diethylamino, Guanidino oder N$'$-Cyanguanidino und

Ar  Phenyl, Methoxyphenyl oder Naphthyl bedeuten;

in Ib

R$^1$  [R$^6$R$^7$N-(CH$_2$)$_m$-NH-CO-(CH$_2$)$_r$]-CH-(Ar-CH$_2$)-CO-,

R$^6$R$^7$N  Amino, Methylamino, Dimethylamino, Ethylamino oder Diethylamino,

m  2 oder 3 und

Ar  Phenyl oder Naphthyl bedeuten;

in Ic

R$^1$  NDP bedeutet;

in Id

Z  Gly, βAla oder His bedeutet;

In Ie

R$^2$  H,

R$^3$  Cyclohexylmethyl,

R$^4$  (H, OH) und

-(CHR$^5$)$_n$- -CH$_2$- oder -CH$_2$CH(Isopropyl)- bedeuten;

in If

-NR$^2$-CHR$^3$-CR$^4$-(CHR$^5$)$_n$-CO- ( = W)  AHCP bedeutet;

in Ig

E  fehlt oder Ile oder Leu bedeutet;

in Ih

R$^1$  (R$^6$R$^7$N-C$_m$H$_{2m}$-NH-CO-CH$_2$)-CH(ArCH$_2$)-CO-, R$^6$R$^7$N-C$_m$H$_{2m}$-N(ArCH$_2$)-CO- oder HO-C$_m$H$_{2m}$-N(ArCH$_2$)-CO-,

R$^6$R$^7$N  Amino, Methylamino, Dimethylamino, Ethylamino oder Diethylamino,

m  2, 3, 4, 5 oder 6,

Ar  Phenyl oder Naphthyl,

R$^2$  H,

R$^3$  Cyclohexylmethyl,

R$^4$  (H, OH)

, -(CHR$^5$)$_n$- -CH$_2$- oder -CH$_2$-CH(Isopropyl)- bedeuten und

E  fehlt oder Ile oder Leu bedeutet;

in Ii

R$^1$  NDP,

Z  Gly, βAla oder His,

R$^2$  H,

R$^3$  Cyclohexylmethyl,

R$^4$  (H, OH),

-(CHR$^5$)$_n$- -CH$_2$- oder -CH$_2$-CH(Isopropyl)- bedeuten

E  fehlt oder Ile bedeutet.

Insbesondere sind bevorzugt Verbindungen der Teilformeln:

I* sowie Ia* bis Ii*, die den Formeln I sowie Ia bis Ii entsprechen, worin jedoch

Q  NH,

Y  H, A oder -CH$_2$R$^{11}$ und

R[11] o-, m- oder p-Aminomethylbenzyl, o-, m- oder p-Guanidinomethylbenzyl, 3-Pyridyl, 4-Amino-2-methyl-5-pyrimidinyl oder 2-Amino-5,6-dimethyl-3-pyrazinyl bedeuten.

I' sowie Ia' bis Ii', die den Formeln I sowie Ia bis Ii entsprechen, worin jedoch

Q NH und

Y H, A, 4-Amino-2-methyl-5-pyrimidinylmethyl oder 2-Amino-5,6-dimethyl-3-pyrazinylmethyl bedeuten.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; ferner EP-A-45665, EP-A-77028, EP-A-77029, EP-A-81783, EP-A-249096) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber an Stelle einer oder mehrerer freier Amino- und/oder Hydroxygruppen entsprechende geschützte Amino- und/oder Hydroxygruppen enthalten, vorzugsweise solche, die an Stelle eines H-Atoms, das mit einem N-Atom verbunden ist, eine Aminoschutzgruppe tragen, z.B. solche die der Formel I entsprechen, aber an Stelle einer His-Gruppe eine N(im)-R'-His-Gruppe (worin R' eine Aminoschutzgruppe bedeutet, z.B. BOM oder DNP) enthalten, oder solche der Formel R[1]-Z-NR[2]-CHR[3]-CH(NHR')-(CHR[5])$_n$-CO-E-Q-Y.

Ferner sind Ausgangsstoffe bevorzugt, die an Stelle des H-Atoms einer Hydroxygruppe eine Hydroxyschutzgruppe tragen, z.B. solche der Formel R[1]-Z-NR[2]-CHR[3]-CHOR''-(CHR[5])$_n$-CO-E-Q-Y, worin R'' eine Hydroxyschutzgruppe bedeutet.

Es können auch mehrere - gleiche oder verschiedene -geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. DNP), Aralkoxymethyl- (z.B. BOM) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, ETOC, 2,2,2-Trichlorethoxycarbonyl, IPOC, BOC, 2-Jodethoxycarbonyl; Aralkyloxycarbonyl wie CBZ, 4-Methoxybenzyloxycarbonyl, FMOC. Bevorzugte Aminoschutzgruppen sind BOC, DNP und BOM, ferner CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäure- und Peptidsynthese hergestellt werden, wie sie z.B. in den genannten Standardwerken und Patentanmeldungen beschrieben sind, z.B. auch nach der Festphasenmethode nach Merrifield.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50$^\circ$, vorzugsweise arbeitet man zwischen 15 und 30$^\circ$ (Raumtemperatur).

Die BOC-Gruppe kann z.B. bevorzugt mit 40%iger Trifluoressigsäure in Dichlormethan oder mit etwa 3 bis 5 n HCl in Dioxan bei 15-30$^\circ$ abgespalten weden, die FMOC-Gruppe mit einer etwa 5-20%iger Lösung von Dimethylamin, Diethylamin oder Piperidin in DMF bei 15-30$^\circ$. Eine Abspaltung der DNP-Gruppe gelingt z.B. auch mit einer etwa 3-10%igen Lösung von 2-Mercaptoethanol in DMF/Wasser bei 15-30$^\circ$.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100$^\circ$ und Drucken zwischen etwa 1 und 200 bar, bevorzugt bei 20-30$^\circ$ und 1-10 bar durchgeführt. Eine Hydrogenolyse der CBZ-Gruppe gelingt z.B. gut an 5-10%igem Pd-C in Methanol bei 20-30$^\circ$.

Verbindungen der Formel I können auch durch direkte Peptidsynthese aus einer Carbonsäure- (Formel II) und einer Aminkomponente (Formel III) erhalten werden. Als Carbonsäurekomponenten eignen sich z. B. solche der Teilformeln R$^1$-OH, R$^1$-Z-OH, R$^1$-Z-W-OH oder R$^1$-Z-W-E-OH als Aminkomponenten solche der Teilformeln H-Z-W-E-Q-Y, H-W-E-Q-Y, H-E-Q-Y oder H-NR$^{10}$-Y. Die Peptidbindung kann aber auch in der Gruppe E geknüpft werden; dabei wird eine Carbonsäure der Formel R$^1$-Z-W-E$^1$-OH mit einer Aminoverbindung der Formel H-E$^2$-Q-Y umgesetzt, wobei E$^1$ + E$^2$ = E ist. Dabei arbeitet man zweckmäßig nach üblichen Methoden der Peptid-Synthese, wie sie z.B. in Houben-Weyl, 1.c., Band 15/II, Seiten 1-806 (1974) beschrieben sind.

Die Reaktion gelingt vorzugsweise in Gegenwart eines Dehydratisierungsmittels, z.B. eines Carbodiimids wie DCCI oder Dimethylaminopropylethyl-carbodiimid, ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, bei Temperaturen zwischen etwa -10 und 40, vorzugsweise zwischen 0 und 30$^\circ$.

An Stelle von II bzw. III können auch geeignete reaktionsfähige Derivate dieser Stoffe in die Reaktion eingesetzt werden, z.B. solche, in denen reaktive Gruppen intermediär durch Schutzgruppen blokkiert sind. Die Carbonsäuren II können z.B. in Form ihrer aktivierten Ester verwendet werden, die zweckmäßig in situ gebildet werden, z.B. durch Zusatz von HOBt oder N-Hydroxysuccinimid.

Die Ausgangsstoffe der Formeln II und III sind großenteils bekannt. Sofern sie nicht bekannt sind, können sie nach bekannten Methoden, z.B. den oben angegebenen Methoden der Peptidsynthese und der Abspaltung von Schutzgruppen hergestellt werden.

Gewünschtenfalls kann in einer Verbindung der Formel I eine funktionell abgewandelte Amino- und/oder Hydroxygruppe durch Solvolyse oder Hydrogenolyse nach einer der oben beschriebenen Methoden in Freiheit gesetzt werden.

So kann z. B. eine Verbindung der Formel I, die eine AcNH-oder eine AOOC-Gruppe enthält, in die entsprechende Verbindung der Formel I umgewandelt werden, die stattdessen eine H$_2$N- oder eine HOOC-Gruppe enthält, zweckmäßig durch selektive Solvolyse nach einer der oben angegebenen Methoden.

Weiterhin können Ketoverbindungen der Formel I (R$^4$ = O) zu Verbindungen der Formel I (R$^4$ = (H, OH)) reduziert werden, beispielsweise mit einem komplexen Metallhydrid wie NaBH$_4$, das nicht gleichzeitig die Peptid-Carbonylgruppen reduziert, in einem inerten Lösungsmittel wie Methanol bei Temperaturen zwischen etwa -10 und +30$^\circ$.

Ketoverbindungen der Formel I (R$^4$ = O) können auch durch reduktive Aminierung in Verbindungen der Formel I (R$^4$ = H, NH$_2$) übergeführt werden. Man kann ein- oder mehrstufig reduktiv aminieren. So kann man z.B. die Ketoverbindung mit Ammoniumsalzen, z.B. Ammoniumacetat, und NaCNBH$_3$ behandeln, vorzugsweise in einem inerten Lösungsmittel, z.B. einem Alkohol wie Methanol, bei Temperaturen zwischen etwa 0 und 50$^\circ$,

insbesondere zwischen 15 und 30°. Weiterhin ist es möglich, die Ketoverbindung zunächst mit Hydroxylamin in üblicher Weise in das Oxim zu überführen und dieses, z.B. durch katalytische Hydrierung an Raney-Nickel, zum Amin zu reduzieren.

Eine Base der Formel I kann mit einer Säure in das zugehörige Säureadditionssalz übergeführt wrden. Für diese Umsetzung kommen insbesondere Säuren in Frage, die physiologisch unbedenkliche Salze liefern, So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z.B. Fluorchlorkohlenwasserstoffen) enthalten. zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine.

Die erfindungsgemäßen Substanzen werden in der Regel in Analogie zu anderen bekannten, im Handel befindlichen Peptiden, insbesondere aber in Analogie zu den in der EP-A-163237, der EP-A-173481 und der EP-A-209897 beschriebenen Verbindungen verabreicht, vorzugsweise in Dosierungen zwischen etwa 100 mg und 10 g, insbesondere zwischen 500 mg und 5 g pro Dosierungseinheit. Die tägliche Dosierung liegt vorzugsweise zwischen etwa 2 und 200 mg/kg, insbesondere 10 und 100 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Renin-abhängige Hypertension und Hyperaldosteronismus können wirksam behandelt werden durch Verabfolgung von Dosierungen zwischen insbesondere etwa 1 und 300, vorzugsweise zwischen 5 und 50 mg/kg Körpergewicht. Für diagnostische Zwecke können die neuen Verbindungen zweckmäßig in Einzeldosen zwischen etwa 0,1 und 10 mg/kg Körpergewicht verabreicht werden.

Vor- und nachstehend sind alle Temperaturen in °C angegeben. In den nachfolgenden Beispielen bedeutet "übliche Aufarbeitung": Man gibt, falls erforderlich, Wasser hinzu, stellt je nach Konstitution des Endprodukts auf pH-Werte zwischen 2 und 8 ein, extrahiert mit Ethylacetat oder Dichlormethan, trennt ab, trocknet die organische Phase über Natriumsulfat, filtriert, dampft ein und reinigt durch Chromatographie an Kieselgel und/oder Kristallisation. Die Angaben "R-Form", "S-Form", "RS-Form" beziehen sich auf das chirale C-Atom im Rest R', insbesondere in der BDP- oder NDP-Gruppe.

### Beispiel 1

Ein Gemisch von 953 mg 2S-Isopropyl-4S-hydroxy-5S-[2-(1-naphthylmethyl)-3-(N-3-dimethylaminopropyl-carbamoyl)-propionyl-N-imi-(2,4-dinitrophenyl)-L-histidyl-amino]-6-cyclohexyl-hexansäure-N-butylamid ["2S-Isopropyl-4S-hydroxy-5S-[NDP-(imi-DNP-His)-amino]-6-cyclohexyl)-hexansäure-N-butylamid"; erhältlich durch Reaktion von 2-(1-Naphthylmethyl)-3-(N-3-dimethylaminopropyl-carbamoyl)-propionsäure ("NDP-OH"; F. 122-123$^•$) mit 2S-Isopropyl-4S-hydroxy-5S-(imi-DNP-His-amino)-6-cyclohexyl-hexansäure-N-butylamid], 2 g 2-Mercaptoethanol, 20 ml DMF und 20 ml Wasser wird unter Rühren bei 20$^•$ mit wässeriger $Na_2CO_3$-Lösung auf pH 8 eingestellt und 2 Std. bei 20$^•$ gerührt. Nach üblicher Aufarbeitung erhält man 2S-Isopropyl-4S-hydroxy-5S-(NDP-His-amino)-6-cyclohexyl-hexansäure-N-butylamid, RS-Form, F. 168-169$^•$.

### Beispiel 2

Man löst 10 g 2S-Isopropyl-4S-hydroxy-5S-[NDP-N-(imi-BOM-His)-amino]-6-cyclohexyl-hexansäure-N-butylamid [erhältlich durch Reaktion von NDP-OH mit 2S-Isopropyl-4S-hydroxy-5S-(imi-BOM-His-amino)-6-cyclohexyl-hexansäure-N-butylamid] in 150 ml Ethanol, hydriert an 5 g 10%ig. Pd-C bei 20$^•$ und 1 bar bis zum Stillstand der $H_2$-Aufnahme, filtriert, dampft ein und erhält nach chromatographischer Reinigung 2S-Isopropyl-4S-hydroxy-5S-(NDP-His-amino)-6-cyclohexyl-hexansäure-N-butylamid, RS-Form, F. 168-169$^•$. R-Form, Dihydrochlorid, F. 178$^•$ (Zers.). S-Form, Dihydrochlorid, F. 145$^•$ (Zers.).

Analog erhält man aus den entsprechenden imi-BOM-His-Derivaten:
BDP-His-AHCP-Ile-OMe
BDP-His-AHCP-Ile-OEt
BDP-His-AHCP-Ile-NH$_2$
BDP-His-AHCP-Ile-N-methylamid
BDP-His-AHCP-Ile-N-ethylamid
BDP-His-AHCP-Ile-N-butylamid
BDP-His-AHCP-Ile-AMPA
BDP-His-AHCP-Ile-ADPA
NDP-His-AHCP-Ile-OMe
NDP-His-AHCP-Ile-OEt
NDP-His-AHCP-Ile-NH$_2$, RS-Form, Dihydrochlorid, F. 209$^•$ (Zers.)
NDP-His-AHCP-Ile-N-methylamid
NDP-His-AHCP-Ile-N-ethylamid
NDP-His-AHCP-Ile-N-butylamid
NDP-His-AHCP-Ile-AMPA
NDP-His-AHCP-Ile-ADPA.

Analog erhält man durch Hydrogenolyse aus 3S-CBZ-amino-4S-NDP-Gly-amino-5-cyclohexyl-pentanoyl-Ile-AMPA das 3S-Amino-4S-NDP-Gly-amino-5-cyclohexyl-pentanoyl-Ile-AMPA ("NDP-Gly-DACP-Ile-AMPA").

Analog sind aus den entsprechenden CBZ-Derivaten herstellbar:
NDP-Gly-DACH-Ile-AMPA
NDP-Gly-DAMH-Ile-AMPA
NDP-Gly-DAPP-Ile-AMPA.

### Beispiel 3

Eine Lösung von 1 g N-Benzyl-N-(6-BOC-aminohexyl)-carbamoyl-Gly-AHCP-Ile-AMPA [F. 135$^•$; erhältlich durch Reaktion von BOC-AHCP-Ile-AMPA (F. 218-220$^•$) mit HCl/Dioxan zu H-AHCP-Ile-AMPA und Umsetzung mit N-Benzyl-N-(6-BOC-aminohexyl)-carbamoyl-Gly-OH] in 20 ml 4 n HCl in Dioxan wird 30 Min. bei 20$^•$ gerührt und dann eingedampft. Man erhält N-Benzyl-N-(6-aminohexyl)-carbamoyl-Gly-AHCP-Ile-AMPA-dihydrochlorid, RS-Form. Freie Base, F. 109$^•$.

Analog erhält man durch Spaltung der entsprechenden N-BOC-Derivate:
N-Benzyl-N-(6-aminohexyl)-carbamoyl-Gly-AHCP-Ile-ADPA
N-Benzyl-N-(6-aminohexyl)-carbamoyl-βAla-AHCP-Ile-AMPA
N-Benzyl-N-(6-aminohexyl)-carbamoyl-βAla-AHCP-Ile-ADPA
2-Benzyl-3-N-(6-aminohexyl)-carbamoyl-propionyl-Gly-AHCP-Ile-AMPA
2-Benzyl-3-N-(6-aminohexyl)-carbamoyl-propionyl-βAla-AHCP-Ile-AMPA
2-Benzyl-3-N-(6-aminohexyl)-carbamoyl-propionyl-Gly-AHCP-Leu-AMPA
2-Benzyl-3-N-(6-aminohexyl)-carbamoyl-propionyl-βAla-AHCP-Leu-AMPA.

Über 2-(1-Naphthylmethyl)-3-N-(6-BOC-aminohexyl)-carbamoyl-propionyl-Gly-OH bzw. -βAla-OH sind erhältlich:
2-(1-Naphthylmethyl)-3-N-(6-aminohexyl)-carbamoyl-propionyl-Gly-AHCP-Ile-AMPA, S-Form, Dihydrochlorid, F. 188$^•$ (Zers.); R-Form, Dihydrochlorid, F. 171$^•$ (Zers.)
2-(1-Naphthylmethyl)-3-N-(6-aminohexyl)-carbamoylpropionyl-βAla-AHCP-Ile-AMPA, RS-Form, Dihydrochlorid, F. 179$^•$ (Zers.)
2-(1-Naphthylmethyl)-3-N-(6-aminohexyl)-carbamoyl-propionyl-βAla-AHCP-Abu-AMPA
2-(1-Naphthylmethyl)-3-N-(6-aminohexyl)-carbamoyl-propionyl-βAla-AHCP-Ala-AMPA
2-(1-Naphthylmethyl)-3-N-(6-aminohexyl)-carbamoyl-propionyl-βAla-AHCP-Cal-AMPA
2-(1-Naphthylmethyl)-3-N-(6-aminohexyl)-carbamoyl-propionyl-βAla-AHCP-Leu-AMPA
2-(1-Naphthylmethyl)-3-N-(6-aminohexyl)-carbamoyl-propionyl-βAla-AHCP-Met-AMPA
2-(1-Naphthylmethyl)-3-N-(6-aminohexyl)-carbamoyl-propionyl-βAla-AHCP-Nle-AMPA
2-(1-Naphthylmethyl)-3-N-(6-aminohexyl)-carbamoyl-propionyl-βAla-AHCP-Nva-AMPA
2-(1-Naphthylmethyl)-3-N-(6-aminohexyl)-carbamoyl-propionyl-βAla-AHCP-Val-AMPA.

Weiterhin erhält man analog:
2-Benzyl-3-N-(8-aminooctyl)-carbamoyl-propionyl-Gly-AHCP-Ile-AMPA
2-Benzyl-3-N-(8-aminooctyl)-carbamoyl-propionyl-βAla-AHCP-Ile-AMPA
2-(1-Naphthylmethyl)-3-N-(8-aminooctyl)-propionyl-carbamoyl-Gly-AHCP-Ile-AMPA
2-(1-Naphthylmethyl)-3-N-(8-aminooctyl)-propionyl-carbamoyl-βAla-AHCP-Ile-AMPA.


Beispiel 4

Eine Lösung von 5,05 g H-Gly-AHCP-Ile-AMPA (erhältlich durch Kondensation von BOC-Gly-AHCP-Ile-OH mit 4-Amino-5-aminomethyl-2-methyl-pyrimidin zu BOC-Gly-AHCP-Ile-AMPA und anschließende Abspaltung der BOC-Gruppe) in 160 ml DMF wird mit 1,01 g N-Methylmorpholin versetzt. Unter Rühren gibt man 3,44 g NDP-OH, 1,35 g HOBt und eine Lösung von 2,06 g DCCI in 50 ml CH$_2$Cl$_2$ hinzu, rührt 12 Std. bei 4°, filtriert den ausgeschiedenen Dicyclohexylharnstoff ab und dampft das Filtrat ein. Nach üblicher Aufarbeitung erhält man NDP-Gly-AHCP-Ile-AMPA, RS-Form, F. 152°; Dihydrochlorid, F. 186° (Zers.). R-Form, F. 142°; Dihydrochlorid, F. 182° (Zers.). S-Form, Dihydrochlorid, F. 178° (Zers.).
Analog erhält man:
BDP-Gly-AHCP-Ile-NH$_2$
BDP-Gly-AHCP-Ile-N-butylamid
BDP-Gly-AHCP-Ile-N-pentylamid
BDP-Gly-AHCP-Ile-N,N-diethylamid
BDP-Gly-AHCP-Ile-AMPA, R-Form, F. 118° (Zers.); S-Form, F. 122° (Zers.)
BDP-Gly-AHCP-Ile-ADPA
NDP-Gly-AHCP-Ile-NH$_2$
NDP-Gly-AHCP-Ile-N-butylamid
NDP-Gly-AHCP-Ile-N-pentylamid
NDP-Gly-AHCP-Ile-N,N-dimethylamid
NDP-Gly-AHCP-Ile-ADPA
2-Benzyl-3-(N-2-dimethylaminoethyl-carbamoyl)-propionyl-Gly-AHCP-Ile-AMPA
2-Benzyl-3-(N-2-dimethylaminoethyl-carbamoyl)-propionyl-βAla-AHCP-Ile-AMPA

2-(1-Naphthylmethyl)-3-(N-2-dimethylaminoethyl-carbamoyl)-propionyl-Gly-AHCP-Ile-AMPA
2-(1-Naphthylmethyl)-3-(N-2-dimethylaminoethyl-carbamoyl)-propionyl-βAla-AHCP-Ile-AMPA
2-Benzyl-3-(N-5-dimethylaminopentyl-carbamoyl)-propionyl-Gly-AHCP-Ile-AMPA
2-Benzyl-3-(N-5-dimethylaminopentyl-carbamoyl)-propionyl-βAla-AHCP-Ile-AMPA
2-(1-Naphthylmethyl)-3-(N-5-dimethylaminopentyl-carbamoyl)-propionyl-Gly-AHCP-Ile-AMPA
2-(1-Naphthylmethyl)-3-(N-5-dimethylaminopentyl-carbamoyl)-propionyl-βAla-AHCP-Ile-AMPA
2-Benzyl-3-(N-6-dimethylaminohexyl-carbamoyl)-propionyl-Gly-AHCP-Ile-AMPA
2-Benzyl-3-(N-6-dimethylaminohexyl-carbamoyl)-propionyl-βAla-AHCP-Ile-AMPA
2-(1-Naphthylmethyl)-3-(N-6-dimethylaminohexyl-carbamoyl)-propionyl-Gly-AHCP-Ile-AMPA
2-(1-Naphthylmethyl)-3-(N-6-dimethylaminohexyl-carbamoyl)-propionyl-βAla-AHCP-Ile-AMPA
2-Benzyl-3-(N-6-guanidinohexyl-carbamoyl)-propionyl-Gly-AHCP-Ile-AMPA
2-Benzyl-3-(N-6-guanidinohexyl-carbamoyl)-propionyl-βAla-AHCP-Ile-AMPA
2-(1-Naphthylmethyl)-3-(N-6-guanidinohexyl-carbamoyl)-propionyl-Gly-AHCP-Ile-AMPA
2-(1-Naphthylmethyl)-3-(N-6-guanidinohexyl-carbamoyl)-propionyl-βAla-AHCP-Ile-AMPA
2-Benzyl-3-[N-6-(N'-cyanguanidino)-hexyl-carbamoyl]-propionyl-Gly-AHCP-Ile-AMPA
2-Benzyl-3-[N-6-(N'-cyanguanidino)-hexyl-carbamoyl]-propionyl-βAla-AHCP-Ile-AMPA
2-(1-Naphthylmethyl)-3-[N-6-(N'-cyanguanidino)-hexyl-carbamoyl]-propionyl-Gly-AHCP-Ile-AMPA
2-(1-Naphthylmethyl)-3-[N-6-(N'-cyanguanidino)-hexyl-carbamoyl]-propionyl-βAla-AHCP-Ile-AMPA
2-Benzyl-4-(N-BOC-N-methyl-amino)-butyryl-βAla-AHCP-Ile-AMPA, RS-Form, Acetat, Rf 0,61 (Kieselgel; 1,1,1-Trichlorethan/Methanol/Essigsäure 75:15:10)
2-Benzyl-5-(N-BOC-N-methyl-amino)-pentanoyl-βAla-AHCP-Ile-AMPA, RS-Form, Acetat, Rf 0,63 (Kieselgel; 1,1,1-Trichlorethan/Methanol/Essigsäure 75:15:10)


Beispiel 5

Analog Beispiel 3 erhält man aus NDP-Gly-OH und H-AHCP-Ile-AMPA das NDP-Gly-AHCP-Ile-AMPA, RS-Form, F. 152°.
Analog erhält man aus N-Benzyl-N-(2-hydroxyethyl)-carbamoyl-Gly-OH (F. 106-107°) das N-Benzyl-N-(2-hydroxyethyl)-carbamoyl-Gly-AHCP-Ile-AMPA (F. 116-118°) und aus N-Benzyl-N-(2-ethoxyethyl)-carbamoyl-Gly-OH das N-Benzyl-N-(2-ethoxyethyl)-carbamoyl-Gly-AHCP-Ile-AMPA (F. 100-102°).

Analog erhält man:
N-Benzyl-N-(6-dimethylamino-hexyl)-carbamoyl-Gly-AHCP-Ile-AMPA
N-Benzyl-N-(6-dimethylamino-hexyl)-carbamoyl-βAla-AHCP-Ile-AMPA
N-(1-Naphthylmethyl)-N-(6-dimethylamino-hexyl)-carbamoyl-Gly-AHCP-Ile-AMPA
N-(1-Naphthylmethyl)-N-(6-dimethylamino-hexyl)-carbamoyl-βAla-AHCP-Ile-AMPA
N-Benzyl-N-(8-dimethylamino-octyl)-carbamoyl-Gly-AHCP-Ile-AMPA
N-Benzyl-N-(8-dimethylamino-octyl)-carbamoyl-βAla-AHCP-Ile-AMPA
N-(1-Naphthylmethyl)-N-(8-dimethylamino-octyl)-carbamoyl-Gly-AHCP-Ile-AMPA
N-(1-Naphthylmethyl)-N-(8-dimethylamino-octyl)-carbamoyl-βAla-AHCP-Ile-AMPA
BDP-Gly-AHCH-Ile-AMPA
BDP-βAla-AHCH-Ile-AMPA
BDP-Gly-Sta-Ile-AMPA
BDP-βAla-Sta-Ile-AMPA
BDP-Gly-AHPP-Ile-AMPA
BDP-βAla-AHPP-Ile-AMPA
NDP-Gly-AHCH-Ile-AMPA
NDP-βAla-AHCH-Ile-AMPA
NDP-Gly-Sta-Ile-AMPA
NDP-βAla-Sta-Ile-AMPA
NDP-Gly-AHPP-Ile-AMPA
NDP-βAla-AHPP-Ile-AMPA.


Beispiel 6

Analog Beispiel 3 erhält man durch Kondensation von NDP-Gly-AHCP-OH mit H-Ile-AMPA das NDP-Gly-AHCP-Ile-AMPA, F. 152˙.
Analog erhält man:
NDP-Gly-AHCP-Abu-AMPA
NDP-Gly-AHCP-Ala-AMPA
NDP-Gly-AHCP-Cal-AMPA
NDP-Gly-AHCP-Leu-AMPA
NDP-Gly-AHCP-Met-AMPA
NDP-Gly-AHCP-Nle-AMPA
NDP-Gly-AHCP-Nva-AMPA
NDP-Gly-AHCP-Phe-AMPA
NDP-Gly-AHCP-Trp-AMPA
NDP-Gly-AHCP-Tyr-AMPA
NDP-Gly-AHCP-Val-AMPA.


Beispiel 7

Analog Beispiel 3 erhält man aus NDP-Gly-AHCP-Ile-OH und H-Ala-AMPA das NDP-Gly-AHCP-Ile-Ala-AMPA.


Beispiel 8

Analog Beispiel 3 erhält man aus NDP-βAla-AHCP-Ile-OH und 4-Amino-5-aminomethyl-2-methyl-pyrimidin das NDP-βAla-AHCP-Ile-AMPA, RS-Form, F. 181˙. Dihydrochlorid, F. 166˙ (Zers.)
Analog erhält man:
BDP-Gly-AHCP-Ile-(N-2-pyridylmethyl-amid)
BDP-βAla-AHCP-Ile-(N-2-pyridylmethyl-amid)
BDP-Gly-AHCP-Ile-(N-3-pyridylmethyl-amid)
BDP-βAla-AHCP-Ile-(N-3-pyridylmethyl-amid)
BDP-Gly-AHCP-Ile-(N-m-aminomethyl-benzyl-amid)
BDP-βAla-AHCP-Ile-(N-m-aminomethyl-benzyl-amid)
BDP-Gly-AHCP-Ile-(N-2-hydroxy-4,6-dimethyl-5-pyridylmethyl-amid)
BDP-βAla-AHCP-Ile-(N-2-hydroxy-4,6-dimethyl-5-pyridylmethyl-amid)
BDP-Gly-AHCP-Ile-(p-sulfamoyl-anilid)
BDP-βAla-AHCP-Ile-(p-sulfamoyl-anilid)
NDP-Gly-AHCP-Ile-(N-2-pyridylmethyl-amid)
NDP-βAla-AHCP-Ile-(N-2-pyridylmethyl-amid)
NDP-Gly-AHCP-Ile-(N-3-pyridylmethyl-amid)
NDP-βAla-AHCP-Ile-(N-3-pyridylmethyl-amid)
NDP-Gly-AHCP-Ile-(N-m-aminomethyl-benzyl-amid)
NDP-βAla-AHCP-Ile-(N-m-aminomethyl-benzyl-amid)
NDP-Gly-AHCP-Ile-(N-2-hydroxy-4,6-dimethyl-5-pyridylmethyl-amid)
NDP-βAla-AHCP-Ile-(N-2-hydroxy-4,6-dimethyl-5-pyridylmethyl-amid)
NDP-Gly-AHCP-Ile-(p-sulfamoyl-anilid)
NDP-βAla-AHCP-Ile-(p-sulfamoyl-anilid).


Beispiel 9

a) Analog Beispiel 3 erhält man aus NDP-Gly-OH und 3-Oxo-4S-amino-5-cyclohexyl-pentanoyl-Ile-AMPA das 3-Oxo-4S-(NDP-Gly-amino)-5-cyclohexyl-pentanoyl-Ile-AMPA.

b) Eine Lösung von 1 g des vorstehenden Keto-amids in 25 ml $CH_3OH$ wird an 0,1 g 10%ig. Pd-C bei 20˙ und 1 bar bis zum Stillstand der $H_2$-Aufnahme hydriert. Nach Filtrieren und Eindampfen erhält man ein Gemisch von 3R- und 3S-Hydroxy-4S-(NDP-Gly-amino)-5-cyclohexyl-pentanoyl-Ile-AMPA.


Beispiel 10

Eine Lösung von 829 mg 3-Oxo-4S-(NDP-Gly-amino)-5-cyclohexylpentanoyl-Ile-AMPA und 1,43 g $Na_2CO_3$ . 10 $H_2O$ in 5 ml Methanol und 5 ml Wasser wird mit 70 mg Hydroxylaminhydrochlorid versetzt und 14 Std. bei 20˙ gerührt. Das ausgefallene Oxim wird abfiltriert, getrocknet. in 10 ml Methanol gelöst und an 0,5 g Raney-Ni bei 20° und 5 bar hydriert. Man filtriert den Katalysator ab, dampft das Filtrat ein, trennt das erhaltene Gemisch an Kieselgel und erhält 3S-Amino-4S-(NDP-Gly-amino)-5-cyclohexyl-pentanoyl-Ile-AMPA ("NDP-Gly-DACP-Ile-AMPA"); daneben erhält man das 3R-Amino-Epimere.

Die nachfolgenden Beispiele betreffen pharmazeutische Zubereitungen.

## Beispiel A: Tabletten

Ein Gemisch von 1 kg NDP-$\beta$Ala-AHCP-Ile-AMPA, 4 kg Lactose, 1,2 kg Maisstärke, 200 g Talk und 100 g Magnesiumstearat wird in üblicher Weise zu Tabletten verpreßt, derart daß jede Tablette 200 mg Wirkstoff enthält.

## Beispiel B: Dragees

Analog Beispiel A werden Tabletten gepreßt, die anschließend in üblicher Weise mit einem Überzug aus Saccharose, Maisstärke, Talk, Tragant und Farbstoff überzogen werden.

## Beispiel C: Kapseln

500 g NDP-Gly-AHCP-Ile-AMPA werden in üblicher Weise in Hartgelatinekapseln gefüllt, so daß jede Kapsel 500 mg Wirkstoff enthält.

## Beispiel D: Injektionsgläser

Eine Lösung von 100 g 2S-Isopropyl-4S-hydroxy-5S-(NDP-His-amino)-6-cyclohexyl-hexansäure-N-butylamid (RS-Form) in 4 l zweifach destilliertem Wasser wird mit 2n Salzsäure auf pH 6,5 eingestellt, steril filtriert und in Injektionsgläser abgefüllt. Man lyophilisiert unter sterilen Bedingungen und verschließt steril. Jedes Injektionsglas enthält 100 mg Wirkstoff.

## Beispiel E: Suppositorien

Man schmilzt ein Gemisch von 50 g NDP-Gly-AHCP-Ile-AMPA mit 10 g Sojalecithin und 140 g Kakaobutter, gießt in Formen und läßt erkalten. Jedes Suppositorium enthält 250 mg Wirkstoff.

## Ansprüche

1. Aminosäurederivate der Formel I

$$R^1\text{-}Z\text{-}NR^2\text{-}CHR^3\text{-}CR^4\text{-}(CHR^5)_n\text{-}CO\text{-}E\text{-}Q\text{-}Y \qquad I$$

worin

$R^1$   $[R^6R^7N\text{-}C_mH_{2m}\text{-}(T)_x\text{-}(CO)_y\text{-}C_rH_{2r}]\text{-}L(R^8\text{-}C_pH_{2p})\text{-}CO\text{-}$ oder $[R^9\text{-}O\text{-}(CO)_z\text{-}C_mH_{2m}\text{-}(T)_x\text{-}(CO)_y\text{-}C_rH_{2r}]\text{-}L(R^8\text{-}C_pH_{2p})\text{-}CO\text{-}$,

$Z$   Abu, Ada, Ala, $\beta$Ala, Arg, Asn, Asp, Bia, Cal, Dab, Ftr, Gln, Glu, Gly, His, Hph, N(im)-A-His, Ile, Leu, tert.-Leu, Lys, Met, $\alpha$Nal, $\beta$Nal, Nbg, Nle, Nva, Orn, Phe, Pia, Pro, Pya, Ser, Thr, Tia, Tic, Trp, Tyr oder Val,

$E$   0 bis 2 peptidartig miteinander verbundene Aminosäurereste ausgewählt aus der Gruppe bestehend aus Abu, Ala, Cal, His, Ile, Leu, Met, Nle, Nva, Phe, Trp, Tyr und Val,

$Q$   O oder $NR^{10}$,

$Y$   $\text{-}C_tH_{2t}\text{-}R^{11}$, $C_tH_{2t}\text{-}R^{12}$ oder $\text{-}C_wH_{2w}\text{-}(CR^{13})_s\text{-}C_tH_{2t}\text{-}R^{11}$,

$T$   O oder $NR^{14}$,

$L$   CH oder N,

$R^2$, $R^5$, $R^9$, $R^{10}$ und $R^{14}$   jeweils H oder A,

$R^3$, $R^8$ und $R^{11}$   jeweils H, A, Ar, Ar-alkyl, Het, Het-alkyl, unsubstituiertes oder ein- oder mehrfach durch A, AO und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

$R^4$ und $R^{13}$   jeweils (H, OH), (H, $NH_2$) oder $=O$,

$R^6$ und $R^7$   jeweils H, A, $\text{-}C(=NH)\text{-}NH_2$ oder $\text{-}C(=NH)\text{-}NHCN$,

$R^6$   auch Ac,

$R^{12}$   $\text{-}SO_3H$, $\text{-}SO_2NH_2$ $\text{-}SO_2NHA$, $\text{-}SO_2NA_2$, $\text{-}NH_2$, $\text{-}NHA$, $\text{-}NA_2$, $\text{-}NH\text{-}C(=NH)\text{-}NH_2$, $\text{-}NH\text{-}C(=NH)\text{-}NHCN$, $\text{-}NH\text{-}CO\text{-}NH_2$, $\text{-}NH\text{-}CO\text{-}NHA$, $\text{-}NH\text{-}CO\text{-}NA_2$, $\text{-}NH\text{-}CS\text{-}NH_2$, $\text{-}NH\text{-}CS\text{-}NHA$ oder $\text{-}NH\text{-}CS\text{-}NA_2$,

$n$ und $s$   jeweils 1 oder 2,

$m$, $p$, $r$, $t$ und $w$   jeweils 0, 1, 2, 3, 4, 5, 6, 7, 8, 9 oder 10,

$x$, $y$ und $z$   jeweils 0 oder 1,

Ar   unsubstituiertes oder ein- oder mehrfach durch A, OA, Hal, $CF_3$, OH, $NO_2$, Hydroxyalkyl, $NH_2$, NHA, $NA_2$, NHAc, SA, SO-A, $SO_2$-A, $SO_2NH_2$ $SO_2NHA$, COOH, COOA, $CONH_2$, CN, Aminoalkyl, HAN-alkyl, $A_2$N-alkyl, $A_3N^{\oplus}$alkyl $An^{\ominus}$ und/oder Guanidinyl-alkyl substituiertes Phenyl oder unsubstituiertes Naphthyl,

Het   einen gesättigten oder ungesättigten 5- oder 6-gliedrigen heterocyclischen Rest mit 1-4 N-, O- und/oder S-Atomen, der mit einem Benzolring kondensiert und/oder ein-oder mehrfach durch A, OA, Hal, $CF_3$, OH, $NO_2$, Carbonylsauerstoff, $NH_2$, NHA, $NA_2$, NHAc, SA, SO-A, $SO_2$-A, $SO_2NH_2$, $SO_2NHA$, COOH, COOA, $CONH_2$, CN, $NH\text{-}SO_2$-A, Ar, Ar-alkyl, Ar-alkenyl, Hydroxyalkyl und/oder Aminoalkyl substituiert sein kann und/oder dessen N- und/oder S-Heteroatome auch oxydiert sein können,

Hal     F, Cl, Br oder J,

Ac     A-CO-, Ar-CO-, Ar-alkyl-CO-, A-O-CO-, Ar-O-CO-, Ar-alkyl-O-CO- oder A-NH-CO-,

$An^{\ominus}$     ein Anion, das auch fehlen kann, wenn stattdessen eine in der Verbindung der Formel I enthaltene Carboxygruppe in Form eines Carboxylatanions vorliegt,

-alkyl-     eine Alkylengruppe mit 1-8 C-Atomen und

A     Alkyl mit 1-8 C-Atomen bedeuten, worin ferner an Stelle einer oder mehrerer -NH-CO-Gruppen auch eine oder mehrere -NA-CO-Gruppen stehen können, sowie deren Salze.

2.     a)     2-(1-Naphthylmethyl)-3-(N-3-dimethylaminopropyl-carbamoyl)-propionyl-βAla-AHCP-Ile-(N-4-amino-2-methyl-5-pyrimidinyl-methyl-amid);

b)     2-(1-Naphthylmethyl)-3-(N-3-dimethylaminopropyl-carbamoyl)-propionyl-Gly-AHCP-Ile-(N-4-amino-2-methyl-5-pyrimidinyl-methyl-amid);

c) 2-Benzyl-3-(N-3-dimethylaminopropyl-carbamoyl)-propionyl-Gly-AHCP-Ile-(N-4-amino-2-methyl-5-pyrimidinyl-methyl-amid);

d) 2-Isopropyl-4-hydroxy-5-[2-(1-naphthylmethyl)-3-(N-3-dimethylaminopropyl-carbamoyl)-propionyl-His-amino]-6-cyclohexyl-hexansäure-N-butyl-amid.

3. Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt oder daß man eine Carbonsäure der Formel II

$R^{1}$-$G^{1}$-OH     II

worin $G^{1}$

(a) fehlt,

(b) Z,

(c) Z-W,

(d) Z-W-$E^{1}$,

(e) Z-W-E und

W     -$NR^{2}$-$CHR^{3}$-$CR^{4}$ $(CHR^{5})_{n}$-CO- bedeuten mit einer Aminoverbindung der Formel III

H-$G^{2}$     III

worin $G^{2}$

(a) -Z-W-E-Q-Y,

(b) -W-E-Q-Y,

(c) -E-Q-Y-,

(d) -$E^{2}$-Q-Y,

(e) -$NR^{1\,\bullet}$-Y und

$E^{1}$ + $E^{2}$     zusammen E bedeuten umsetzt,

und daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder zur Herstellung einer Verbindung der Formel I, $R^{4}$ = (H, OH) oder (H, $NH_{2}$), ein Aminoketosäurederivat der Formel I, $R^{4}$ = O, reduziert oder reduktiv aminiert und/oder eine Verbindung der Formel I durch Behandeln mit einer Säure in eines ihrer Salze überführt.

4. Verfahren zur Herstellung pharmazeutischer Zubereitungen, dadurch gekennzeichnet, daß man eine Verbindung der Formel I und/oder eines ihres physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verwendung von Verbindungen der Formel I oder von deren physiologisch unbedenklichen Salzen zur Herstellung eines Arzneimittels.

7. Verwendung von Verbindungen der Formel I oder von deren physiologisch unbedenklichen Salzen bei der Bekämpfung der reninabhängigen Hypertension oder des Hyperaldosteronismus.